(19) Europäisches Patentamt
European Patent Office
Office européen des brevets

(11) Publication number: **0 103 417**
**B1**

(12) **EUROPEAN PATENT SPECIFICATION**

(45) Date of publication of patent specification: **29.10.86**

(51) Int. Cl.⁴: **C 07 D 471/04,** A 61 K 31/435

(21) Application number: **83304702.0**

(22) Date of filing: **15.08.83**

(54) **3-Deazaguanine sulphonic acid salts.**

(30) Priority: **19.08.82 US 409389**

(43) Date of publication of application:
**21.03.84 Bulletin 84/12**

(45) Publication of the grant of the patent:
**29.10.86 Bulletin 86/44**

(84) Designated Contracting States:
**AT BE CH DE FR GB IT LI LU NL SE**

(58) References cited:
**EP-A-0 082 369**
**DD-A- 124 251**
**US-A-3 896 135**
**US-A-4 322 411**

**JOURNAL OF THE AMERICAN CHEMICAL SOCIETY, Vol. 98, March 17, 1976, American Chemical Society, Books and Journals Division P. DAN COOK et al. "Synthesis of 3-Deazaguanine, 3-Deazaguanosine, and 3-Deazaguanylic Acid by a Novel Ring Closure of Imidazole Precursors", pages 1492-1498**

(73) Proprietor: **WARNER-LAMBERT COMPANY**
**201 Tabor Road**
**Morris Plains New Jersey 07950 (US)**

(72) Inventor: **Pattison, Ian C.**
**3460 Brentwood Ct.**
**Ann Arbor, MI 48105 (US)**

(74) Representative: **Jones, Michael Raymond et al**
**HASELTINE LAKE & CO. Hazlitt House**
**28 Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 103 417 B1

**Description**

This invention relates to novel water-soluble sulphonic acid salts of 3-deazaguanine.

At some parts in the specification there are references to a radical $R_1$, which is the same as the radical $R^1$ referred to elsewhere.

The abbreviations Ac, Me and Et appearing hereinbelow represent acetyl, methyl and ethyl, respectively.

The abbreviation q.s. appearing hereinbelow means "as required (up to the desired total volume)".

The compound 6-amino-1,5-dihydro-4*H*-imidazo[4,5-c]pyridin-4-one (3-deazaguanine) is described and claimed in US Patent 3,896,135. The patent describes the compound as possessing antiviral and antibacterial activity. More recent reports have described the compound as also possessing antitumor activity J. Am. Chem. Soc. *98,* 1492 (1976). Drug administration by the parenteral route, especially the intravenous route, for this last indication is preferred.

The US Patent also discloses that 3-deazaguanine forms salts with bases such as sodium hydroxide and with acids such as hydrochloric and sulfuric. Unfortunately, investigation has shown that these salts are not suitable for preparing parenteral dosage forms. Thus, the sodium salt solution possesses a pH at which 3-deazaguanine is unstable. The hydrochloride and sulfate salts are poorly soluble so that administration of aqueous parenteral solutions of these salts is impractical. In fact, it has been found that the sulfate salt is only ca. 1/2 as soluble as the corresponding free base. This observation is quite surprising and is the reverse of the characteristic increase in solubility of acid addition salts of basic compounds.

The present invention provides certain substituted sulfonic acid salts of 3-deazaguanine. These salts are unexpectedly highly water soluble and may be utilized to produce convenient pharmaceutical dosage forms. In particular, the salts may be utilised to produce dosage forms which are suitable for parenteral administration. The parenteral dosage forms may be in the form of solutions, suspensions or dry solids which may be reconstituted with a suitable liquid prior to administration.

According to one aspect of the present invention, there is provided a compound having the structural formula I

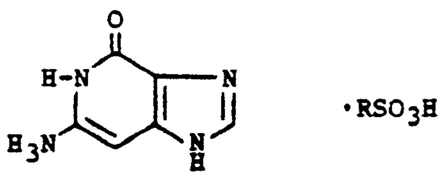

I

wherein R is phenyl or $R_1CH_2$ wherein $R_1$ is a hydrogen atom or an alkyl radical having one to three carbon atoms optionally substituted with from one to three hydroxyl groups.

Preferably, $R_1$ is a hydrogen atom or an unsubstituted radical having from one to three carbon atoms. $R_1$ may also be an alkyl radical having from one to three carbon atoms which is substituted with from one to three hydroxyl group(s).

Particularly preferred compounds are those in which $R_1$ is hydrogen; $R_1$ is methyl; $R_1$ is hydroxy-methyl; and R is phenyl.

According to a second aspect of the present invention, there is provided a pharmaceutical composition which is a stable, solid, water-soluble composition suitable for reconstitution with a pharmaceutically acceptable solvent, useful for the preparation of a stable solution containing the equivalent of at least l5 mg/ml of 3-deazaguanine free base, said composition being prepared by the steps of:

1) preparing a solution of a compound having the structural formula

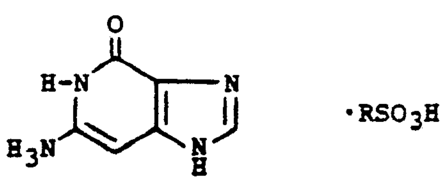

I

wherein R is defined hereinabove; and

2) removing the solvent from the so prepared solution.

The free base, 6-amino-1,5-dihydro-4*H*-imidazo[4,5-c]pyridin-4-one, 3-deazaguanine, may be prepared by procedures described in US Patent 3,896,135.

The substituted sulfonic acids utilized to prepare the final compounds of the invention are either commercially available or they may be prepared by standard procedures known in the art.

The salts of the invention may be prepared by standard procedures. Thus, approximately equal equivalent amounts of 3-deazaguanine base and the desired salt forming substituted sulfonic acid are combined in a convenient volume of a solvent such as water to obtain complete solution. The solvent may

be removed, for example by lyophilization, and the residue may be purified if desired. For example the sale may be recrystallized. Alternatively, approximate equal equivalent amounts of 3-deazaguanine and the desired acid may be combined in a minimum volume of solvent to produce a suspension. The suspension may be moderately heated if desired to promote complete solution.

The so produced salt may be isolated and purified as described above. Solvents other than water may be utilized for the preparation of the salt. Examples of such solvents are alcohols such as methanol, ethanol, propanol, and the like; ketones such propanone, butanone, and the like; *N,N*-dimethylformamide; dimethylsulfoxide as well as mixtures thereof.

For preparing individual sterile dosage forms, the appropriate volume of solution containing a compound of the invention may be sterile filtered into previously sterilized containers such as vials. The solvent may be removed by distillation or lyophilization and the containers may be closed as by sealing or capping. The so produced dosage forms may be reconstituted with an appropriate solvent or suspending medium prior to use. Such appropriate solvents or media are exemplified by pharmaceutically acceptable solvents or mixtures thereof such as distilled water, sterile water, sterile isotonic media such as glucose solution, ethanol, ethylene glycol, propylene glycol, and the like; as well as mixtures thereof. The reconstituted dosage form may be in the form of a solution such that would be useful for intravenous administration for example, or in the form of a suspension useful for intramuscular administration.

Similar individual sterile dosage forms may also be prepared by sterile filling appropriate containers with a compound of the invention which is in dry solid form. Procedures for performing the dry sterile fill operation will be familiar to those skilled in the art.

The compounds of the invention may also be utilized to prepare other dosage forms. For example tablets, capsules, powders, and suppositories may be prepared by standard methods.

The alkyl groups contemplated by the invention designated by the symbol $R_1$ may be both branched and straight chain of from one to three carbon atoms. Examples of such groups are methyl, ethyl, propyl and *i*-propyl.

The alkyl groups, in addition, may be substituted with one or more hydroxyl groups. Those skilled in the art will recognize that only one hydroxyl group may be substituted on any particular carbon atom. The hydroxylated alkyl groups therefore contain no more than three hydroxyl groups, each located on different carbon atoms of the chain. Examples of the preferred hydroxylated alkyl groups are $HOCH_2$, $HOCH_2CH_2$,

$$CH_3\underset{\substack{|\\ OH}}{CH}—,\quad HOCH_2\underset{\substack{|\\ OH}}{CH}—CH_2,$$

and the like.

A preferred specie of the invention is that wherein $R_1$ is hydrogen, i.e., 3-deazaguanine methanesulfonic acid salt.

Certain alkyl groups of the invention may possess an asymmetric carbon atom and therefore optical isomers may be possible. The pure isomers as well as mixtures thereof including racemic mixtures are contemplated by the invention.

The compounds of the invention may exist in unsolvated as well as solvated forms, including hydrated forms. In general, the solvated forms, with pharmaceutically acceptable solvents such as water, ethanol, and the like are equivalent to the unsolvated forms for purposes of the invention.

The following procedure was utilized to determine the solubilities of various salts of 3-deazaguanine. The free base of 3-deazaguanine (250 mg) was suspended in a small volume of water and one equivalent weight of the desired acid was added with stirring. Stirring was continued and additional volumes of water were added intermittently until complete solution was obtained. Utilizing this procedure the results in the following table were obtained.

Solubilities of 3-Deazaguanine Salts

| Acid | ml $H_2O$ needed to Dissolve | Solubility mg/ml | Relative Solubility of Salt to Free Base* |
|---|---|---|---|
| Free Base | 100—125 | 2.0—2.5 | — |
| HCl | 65—75 | 3.3—3.8 | 1.9 |
| $H_2SO_4$ | 250—300 | 0.8—1.0 | 0.5 |
| $H_3PO_4$ | 120—130 | 1.9—2.1 | 1.05 |
| HOAc | 85—95 | 2.6—2.9 | 1.45 |
| Citric | 170—180 | 1.4—1.5 | 0.75 |
| Maleic | 150—160 | 1.6—1.7 | 0.85 |
| d-Tartaric | 150—160 | 1.6—1.7 | 0.85 |
| $MeSO_3H$ | 5 | 50 | 25 |
| $HO(CH_2)_2SO_3H$ | 5—10 | 25—50 | 25 |
| $EtSO_3H$ | 10 | 25 | 12.5 |
| $C_6H_5SO_3H$ | 25 | 10 | 5.0 |

*These values were obtained by dividing the higher value of the solubility range for the particular salt by the 2.0 mg/ml solubility of the free base.

The results in the table demonstrate the solubility advantage for the compounds of the invention which advantage is particularly surprising in view of the decreased water solubility of the sulfate salt when compared to the free base.

Example I

To a slurry of 24.5 g of 5-amino-1,5-dihydro-4*H*-imidazo[4,5-*c*]pyridin-4-one (3-deazaguanine) in 250 ml methyl alcohol was added a solution of 17.0 g of methanesulfonic acid in 50 ml of methyl alcohol. The mixture was stirred for about 10 minutes at room temperature, filtered, and the filter cake washed with methyl alcohol to give, after drying 35.5 g (88%) of 3-deazaguanine methanesulfonate salt, mp 273—275°C. This material produced a solution in water at a concentration of 41 mg/ml which remained clear for more than three days.

A 1.0 g sample of this salt was recrystallized from 22 ml of 80% aqueous methyl alcohol to give material melting at 281—283°C.

PHARMACEUTICAL COMPOSITIONS
EXAMPLE 1

| Ingredient | Quantity |
|---|---|
| 3-Deazaguanine methane-sulfonic acid salt | 300 g |
| Lactose | 974 g |
| Corn Starch | 39 g |
| Hydroxypropyl cellulose | 30 g |
| Magnesium stearate | 7 g |
| Ethanol-water 50:50 | qs |

The 3-Deazaguanine methanesulfonic acid salt lactose, and hydroxypropyl cellulose are blended and granulated with 50:50 ethanol water. The wet granulation is screened, dried, and rescreened. The resulting dried granulation is blended with magnesium stearate and the corn starch and the mixture is compressed into 450 mg tablets using an 11/32 inch standard concave punch. Yield equals approximately 3000 tablets each containing 100.0 mg of 3-Deazaguanine methanesulfonic acid salt.

EXAMPLE 2

| Ingredient | Quantity |
|---|---|
| 3-Deazaguanine methane-sulfonic acid salt | 1000 g |
| Lactose | 1900 g |
| Corn Starch | 50 g |
| Hydroxypropyl cellulose | 40 g |
| Magnesium stearate | 10 g |
| Ethanol-water 50:50 | qs |

The 3-Deazaguanine methanesulfonic acid salt lactose, and hydroxypropyl cellulose are blended and granulated with 50:50 ethanol water. The wet granulation is screened, dried, and rescreened. The resulting dried granulation is blended with magnesium stearate and the corn starch and the mixture is compressed into 750 mg tablets using a standard concave punch. Yield equals approximately 4000 tablets each containing 250.0 mg of 3-Deazaguanine methanesulfonic acid salt.

EXAMPLE 3

| Ingredient | Quantity |
|---|---|
| 3-Deazaguanine methane-sulfonic acid salt | 500 g |
| Lactose | 1473 g |
| Magnesium stearate | 27 g |

The mixture is blended and filled into hard gelatin capsules, filling each capsule with 400 mg of the powder mixture. Yield equals approximately 5,000 capsules each containing 100.0 mg of 3-Deazaguanine methanesulfonic acid salt.

EXAMPLE 3

| Ingredient | Quantity |
|---|---|
| 3-Deazaguanine methane-sulfonic acid salt | 1000 g |
| Lactose | 1950 g |
| Magnesium stearate | 50 g |

The mixture is blended and filled with hard gelatin capsules, filling each capsule with 750 mg of the powder mixture. Yield equals approximately 4,000 capsules each containing 250.0 mg of 3-Deazaguanine methanesulfonic acid salt.

EXAMPLE 4

| Ingredient | Quantity |
|---|---|
| 1. Methanesulfonic Acid, 98% Solution | 18.2 g |
| 2. 3-Deazaguanine | 25.0 g |
| 3. Water for Injection | q.s. ad 1000 ml |
| 4. Nitrogen Gas High Purity | q.s. |

Method of Preparation

In a suitable container, mix 1 with approximately 725 ml of 3, to yield A; Add 2 to A with mixing until 2 is dissolved; Add a sufficient amount of 3 to make 1000 ml of solution and mix well;

Sterilize the solution by filtering through appropriate prefilters followed by filtration through a previously sterilized membrane, using 4 for positive pressure; Aseptically fill 10.0 ml of the solution into previously sterilized vials; stopper loosely with lyophilization closures and lyophilize; Cap the lyophilized vials.

When reconstituted with 10.0 ml of Sterile Water for Injection, the resultant solution will contain 25 mg/ml of 3-deazaguanine as the methanesulfonic acid salt.

**Claims for the Contracting States: BE CH DE FR GB IT LI LU NL SE**

1. A compound having the following structural formula

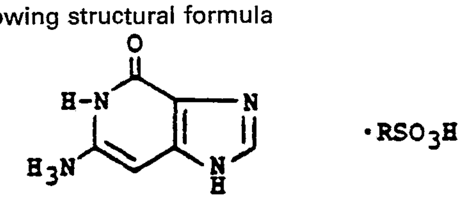

I

wherein R is phenyl or $R^1CH_2$, wherein $R^1$ is a hydrogen atom or an alkyl radical having from one to three carbon atoms which is optionally substituted with from one to three hydroxyl group(s).

2. A compound as claimed in Claim 1, wherein $R^1$ is a hydrogen atom or an unsubstituted alkyl radical having from one to three carbon atoms.

3. A compound as claimed in Claim 1, wherein $R^1$ is hydrogen, the compound being 6-amino-1,5-dihydro-4*H*-imidazo[4,5-c]pyridin-4-one methanesulphonic acid salt.

4. A compound as claimed in Claim 1, wherein $R^1$ is $CH_3$, the compound being 6-amino-1,5-dihydro-4*H*-imidazo[4,5-c]pyridin-4-one ethanesulphonic acid salt.

5. A compound as claimed in Claim 1, wherein $R^1$ is an alkyl radical having from one to three carbon atoms substituted with up to three hydroxyl groups.

6. A compound as claimed in Claim 5, wherein $R^1$ is $HOCH_2$.

7. A compound as claimed in Claim 1, wherein R is a phenyl radical.

8. A stable, solid, water-soluble composition suitable for reconstitution, useful for the preparation of a stable solution containing the equivalent of at least 15 mg/ml of 3-deazaguanine free base, the composition being prepared by the steps of

(a) preparing a solution in a solvent of a compound as claimed in Claim 1; and

(b) removing the solvent from the thus prepared solution.

9. A composition as claimed in Claim 8, which comprises 3-deazaguanine methanesulphonic acid salt.

10. A composition as claimed in Claim 8 or 9, which is reconstituted with a pharmaceutically acceptable solvent.

**Claims for the Contracting State: AT**

1. A process for producing a compound having the following structural formula I

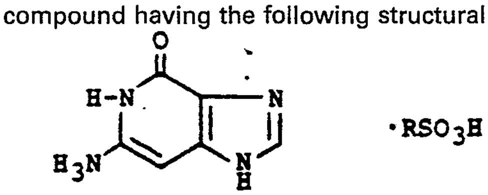

I

wherein R is phenyl or $R^1CH_2$, wherein $R^1$ is a hydrogen atom or an alkyl radical having from one to three carbon atoms optionally substituted with from one to three hydroxyl groups; which process comprises the steps of

(a) preparing a solution of a compound of formula I by mixing in a solvent 3-deazaguanine and $RSO_3H$ where R is as defined above; and

(b) removing the solvent from the thus prepared solution.

2. A process according to Claim 1, wherein $R^1$ is a hydrogen atom or an unsubstituted alkyl radical having from one to three carbon atoms.

3. A process according to Claim 1, wherein $R^1$ is hydrogen, for producing 6-amino-1,5-dihydro-4*H*-imidazo[4,5-c]pyridin-4-one methanesulphonic acid salt.

4. A process according to Claim 1, wherein $R^1$ is $CH_3$, the compound being 6-amino-1,5-dihydro-4*H*-imidazo[4,5-c]-pyridin-4-one ethanesulphonic acid salt.

5. A process according to Claim 1, wherein $R^1$ is an alkyl radical having from one to three carbon atoms substituted with up to three hydroxyl groups.

6. A process according to Claim 1 wherein $R^1$ is $HOCH_2$—.

7. A process according to Claim 1, wherein R is a phenyl.

8. A process according to any preceding claim, which further includes reconstituting a liquid preparation, with a pharmaceutically acceptable solvent.

**Patentansprüche für die Vertragsstaaten: BE CH DE FR GB IT LI LU NL SE**

1. Verbindung mit der folgenden Strukturformel

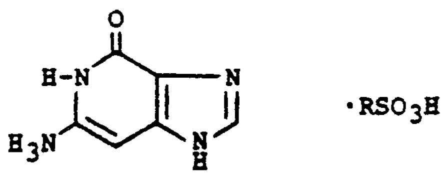

I

worin R für Phenyl oder $R^1CH_2$ steht, wobei $R^1$ ein Wasserstoffatom oder ein gewünschtenfalls mit 1 bis 3 Hydroxylgruppe(n) substituiertes Alkylradikal mit 1 bis 3 Kohlenstoffatomen ist.

2. Verbindung nach Anspruch 1, worin $R^1$ ein Wasserstoffatomen oder ein unsubstituiertes Alkylradikal mit 1 bis 3 Kohlenstoffatomen darstellt.

3. Verbindung nach Anspruch 1, worin $R^1$ für Wasserstoff steht, welche Verbindung 6-Amino-1,5-dihydro-4H-imidazo[4,5-c]pyridin-4-on-methansulfonsäuresalz ist.

4. Verbindung nach Anspruch 1, worin $R^1CH_3$ repräsentiert, welche Verbindung 6-Amino-1,5-dihydro-4H-imidazo[4,5-c]pyridin-4-on-äthansulfonsäuresalz ist.

5. Verbindung nach Anspruch 1, worin $R^1$ ein mit bis zu 3 Hydroxylgruppen substituiertes Alkylradikal mit 1 bis 3 Kohlenstoffatomen ist.

6. Verbindung nach Anspruch 5, worin $R^1$ für $HOCH_2$ steht.

7. Verbindung nach Anspruch 1, worin $R^1$ für ein Phenylradikal steht.

8. Stabile, feste, wasserlösliche zur Rekonstitution geeignete, zur Herstellung einer die äquivalente Menge von wenigstens 15 mg/ml freie 3-Deazaguaninbase enthaltenden stabilen Lösung nützliche Zusammensetzung, welche Zusammensetzung mittels der folgenden Schritte hergestellt wird:

(a) Herstellung einer Lösung einer Verbindung nach Anspruch 1 in einem Lösungsmittel; und

(b) Entfernung des Lösungsmittels aus der so hergestellten Lösung.

9. Zusammensetzung nach Anspruch 8, welche 3-Deazaguaninmethansulfonsäuresalz umfaßt.

10. Zusammensetzung nach Anspruch 8 oder 9, welche mit einem pharmazeutisch akzeptablem Lösungsmittel rekonstituiert wird.

**Patentansprüche für den Vertragsstaat: AT**

1. Verfahren zur Herstellung einer Verbindung mit der folgenden Strukturformel I

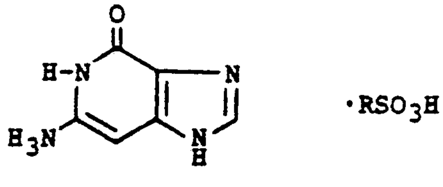

I

worin R für Phenyl oder $R^1CH_2$ steht, wobei $R^1$ ein Wasserstoffatom oder ein gewünschtenfalls mit 1 bis 3

Hydroxylgruppe(n) substituiertes Alkylradikal mit 1 bis 3 Kohlenstoffatomen ist; welches Verfahren dadurch gekennzeichnet ist, daß es die folgenden Schritte umfaßt:

(a) Herstellung einer Lösung einer Verbindung der Formel I durch Mischen von 3-Deazaguanin und $RSO_3H$ in einem Lösungsmittel, wobei R wie oben definiert ist; und

(b) Entfernen des Lösungsmittels aus der so hergestellten Lösung.

2. Verfahren nach Anspruch 1, worin $R^1$ für ein Wasserstoffatom oder ein unsubstituiertes Alkylradikal mit 1 bis 3 Kohlenstoffatomen steht.

3. Verfahren nach Anspruch 1, worin $R^1$ Wasserstoff darstellt, zur Herstellung 6-Amino-1,5-dihydro-4H-imidazo[4,5-c]pyridin-4-on-methansulfonsäuresalz.

4. Verfahren nach Anspruch 1, worin $R^1$ für $CH_3$ steht, welche Verbindung 6-Amino-1,5-dihydro-4H-imidazo[4,5-c]pyridin-4-on-äthansulfonsäuresalz ist.

5. Verfahren nach Anspruch 1, worin $R^1$ ein mit bis zu 3 Hydroxylgruppen substituiertes Alkylradikal mit 1 bis 3 Kohlenstoffatomen repräsentiert.

6. Verfahren nach Anspruch 1, worin $R^1$ $HOCH_2$— ist.

7. Verfahren nach Anspruch 1, worin $R^1$ für Phenyl steht.

8. Verfahren nach irgendeinem der vorhergehenden Ansprüche, dadurch gekennzeichnet, daß es weiters die Rekonstitution eines flüssigen Präparates mit einem pharmazeutisch akzeptablen Lösungsmittel einschließt.

**Revendications pour les Etats contractants: BE CH DE FR GB IT LI LU NL SE**

1. Un composé de formule structurelle:

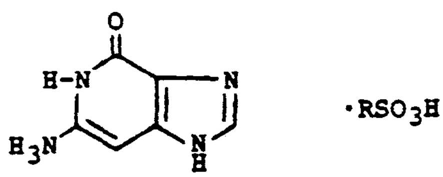

I

dans laquelle:

R représente un phényle ou $R_1CH_2$ dans lequel $R_1$ représente un atome d'hydrogène ou un radical alkyle possédant de 1 à 3 atomes de carbone, éventuellement substitués par 1 à 3 groupes hydroxyles.

2. Un composé selon la revendication 1, dans lequel $R^1$ représente un atome d'hydrogène ou un radical alkyle non-substitué possédant de 1 à 3 atomes de carbone.

3. Un composé selon la revendication 1, dans lequel $R^1$ représente un atome d'hydrogène, le composé étant le sel de l'acide méthane sulfonique de 6-amino-1,5-dihydro-4H-imidazo-{4,5-c}-pyridine-4-one.

4. Un composé selon la revendication 1, dans lequel $R^1$ représente $CH_3$, le composé étant le sel de l'acide éthane sulfonique de 6-amino-1,5-dihydro-4H-imidazo-{4,5-c}-pyridine-4-one.

5. Un composé selon la revendication 1, dans lequel $R^1$ représente un radical alkyle possédant de 1 à 3 atomes de carbone substitués par jusqu'à trois groupes hydroxyles.

6. Un composé selon la revendication 5, dans lequel $R^1$ représente $HOCH_2$.

7. Un composé selon la revendication 5, dans lequel $R^1$ représente un radical phényle.

8. Une composition stable solide soluble dans l'eau convenant à la reconstitution utilisable pour la préparation d'une solution stable contenant l'équivalent d'au moins 15 mg par ml de la base libre déaza-3-guanine, cette composition étant préparée par les étapes consistant à:

a. — préparer une solution dans un solvant d'un composé selon la revendication 1, et

b. — éliminer le solvant de la solution ainsi préparée.

9. Une composition selon la revendication 8 qui est constituée par le méthane sulfonate de déaza-3-guanine.

10. Une composition selon la revendication 8 ou 9 qui est constituée avec un solvant pharmaceutiquement acceptable.

**Revendications pour l'Etat contractant: AT**

1. Un procédé de préparation d'un produit de formule structurelle I suivante:

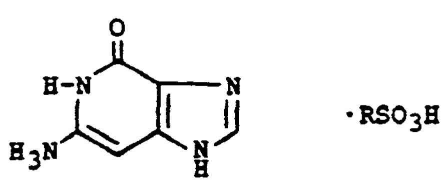

I

dans laquelle

R représente un phényle ou $R_1CH_2$ dans lequel $R_1$ représente un atome d'hydrogène ou un radical alkyle possédant de 1 à 3 atomes de carbone, éventuellement substitués par 1 à 3 groupes hydroxyles; ce procédé comprenant les étapes consistant à:

a. — préparer une solution d'un composé de formule I par mélange dans un solvant de déaza-3-guanine et de $RSO_3H$ avec R étant tel que défini ci-dessus; et

b. — éliminer le solvant de la solution ainsi préparée.

2. Un procédé selon la revendication 1, dans lequel $R^1$ représente un atome d'hydrogène ou un radical alkyle non-substitué possédant de 1 à 3 atomes de carbone.

3. Un procédé selon la revendication 1, dans lequel $R^1$ représente un atome d'hydrogène pour préparer le sel de l'acide méthane sulfonique de 6-amino-1,5-dihydro-4H-imidazo-{4,5-c}-pyridine-4-one.

4. Un procédé selon la revendication 1, dans lequel $R^1$ représente $CH_3$, le composé étant le sel de l'acide éthane sulfonique de 6-amino-1,5-dihydro-4H-imidazo-{4,5-c}-pyridine-4-one.

5. Un procédé selon la revendication 1, dans lequel $R^1$ est un radical alkyle possédant de 1 à 3 atomes de carbone substitués par jusqu'à 3 groupes hydroxyles.

6. Un procédé selon la revendication 1, dans lequel $R^1$ représente $HOCH_2$—.

7. Un procédé selon la revendication 1, dans lequel R représente un radical phényle.

8. Un procédé selon l'une quelconque des revendications précédentes comprenant, en outre, la reconstitution d'une préparation liquide à l'aide d'un solvant pharmaceutiquement acceptable.